# EUROPÄISCHE PATENTSCHRIFT

(11) **EP 2 012 868 B1**
(45) Veröffentlichungstag und Bekanntmachung des Hinweises auf die Patenterteilung: **20.07.2011**
(21) Anmeldenummer: 07724803.7
(22) Anmeldetag: 02.05.2007
(51) Int. Cl.: A61M 39/14

(54) **VORRICHTUNG UND VERFAHREN ZUM ABGESCHLOSSENEN, TROPFFREIEN UND SICHEREN TRANSFER VON FLUIDEN**
SYSTEM AND METHOD FOR CLOSED, DRIP-FREE AND SECURE TRANSFER OF FLUIDS
DISPOSITIF ET PROCÉDÉ POUR LE TRANSFERT FERMÉ, SÛR ET SANS FUITE DE FLUIDES

(30) Priorität: 04.05.2006 DE 102006020845
(43) Veröffentlichungstag der Anmeldung: 14.01.2009
(73) Patentinhaber: Isotopen Technologien München AG, 85748 Garching (DE)
(72) Erfinder: SCHILP, Michael, 85748 Garching (DE); BUCK, Oliver, 83457 Bayerisch Gmain (DE)
(74) Vertreter: Hartig, Michael
(86) Internationale Anmeldenummer: PCT/EP2007/003877
(87) Internationale Veröffentlichungsnummer: WO 2007/128481

(56) Entgegenhaltungen:
- EP-A- 0 126 718
- EP-A- 0 584 396
- FR-A1- 2 696 526
- US-A- 5 122 123

## Beschreibung

Die Erfindung betrifft ein für eine Verwendung im medizinischen Bereich geeignetes Anschlußstück zum abgeschlossenen, tropffreien und sicheren Transfer von Fluiden sowie eine zwei Anschlußstücke umfassende Vorrichtung, bei der ein Austritt und eine Verunreinigung eines Fluids sowohl bei miteinander verbundenen Anschlußstücken als auch nach dem Trennen der Anschlußstücke vermieden werden kann und ein entsprechendes Verfahren.

Bei der Herstellung und Verabreichung von Medikamenten müssen Ausgangssubstanzen und hergestellte Medikamente zwischen verschiedenen Vorrichtungen, wie beispielsweise Behältern, Dosierungs- und Mischvorrichtungen und Einrichtungen zur Verabreichung, wie beispielsweise Spritzen oder Kathetern transferiert werden. Sofern aufgrund der Substanzen ein Gesundheitsrisiko für die damit umgehenden Personen besteht, muß eine Berührung bzw. Kontaminierung in jedem Fall vermieden werden. Dies gilt insbesondere für gefährliche Substanzen einschließlich von Säuren und radioaktiven Stoffen, wie beispielsweise mit radioaktiven Isotopen markierte Medikamente oder Substanzverbindungen, deren Herstellung aufgrund ihrer zeitlichen Veränderlichkeit nur in der Nähe des Verabreichungsorts und relativ kurzfristig vor der Verabreichung erfolgen kann.

Andererseits ist es zur Sicherstellung einer hohen Produktqualität wünschenswert, eine Verunreinigung von Medikamenten und der Ausgangssubstanzen bei der Herstellung und auch nach der Herstellung, wie beispielsweise beim Transfer in Einrichtungen zur Dosierung, Aufbewahrung und Verabreichung, sowie bei der Verabreichung selbst zu vermeiden.

Im Stand der Technik sind verschiedene Einrichtungen bekannt, die bei der manuellen, halbautomatischen oder vollständig automatisierten Herstellung zum Transfer, Dosieren, Mischen und Verabreichen von unter anderem gefährliche, insbesondere giftige oder radioaktive Stoffe enthaltenden Medikamenten oder Substanzverbindungen verwendet werden. Beispielsweise werden sogenannte Luer-Locks zur Verbindung offener Schläuche oder von Schläuchen, Kathetern oder Nadeln verwendet, die gegebenenfalls mit einem zusätzlichen Sperrhahn oder Deckel abschließbar sind. Obwohl mit Luer-Lock-Komponenten versehene Schläuche miteinander oder mit Kathetern, Nadeln usw. verschraubt werden können, so daß eine dichte Verbindung hergestellt und ein unbeabsichtigtes Lösen ausgeschlossen werden kann, besteht der Nachteil, daß beim Trennen der miteinander verbundenen Komponenten Tropfen entstehen und Personen damit in Berührung kommen können, was bei gefährlichen Substanzen Risiken beinhaltet.

Des weiteren ist im Stand der Technik die Verwendung eines Septums und einer Nadel zum Dosieren und Transferieren von Substanzen bekannt. Nachteilig ist jedoch, daß das Einstechen der Nadel in das Septum einen Überdruck und eine Tropfenbildung auslösen kann und daß nach der Entnahme der Nadel aus einem mit dem Septum versehenen Behälter gegebenenfalls Tropfen aus der Nadel entweichen und mit Personen in Kontakt kommen können. Des weiteren besteht aufgrund der in der Regel scharfen Spitze der Nadel eine Verletzungsgefahr.

Als eine gängige Verbindung zwischen Schläuchen zum Transfer von in erster Linie gasförmigen Fluiden sind des weiteren Kupplungen bekannt, die sich beim Einstecken eines Gegenstücks selbsttätig öffnen und beim Entfernen des Gegenstücks automatisch schließen. Jedoch wird in der Regel nur die mit Druck beaufschlagte Seite verschlossen, und es besteht kein Schutz gegen Nachtropfen. Darüber hinaus ist eine Kontamination durch Kontakt mit der Außenseite der getrennten Schlauch- bzw. Leitungsabschnitte möglich.

Die genannten Vorrichtungen bieten auch einen gewissen Schutz vor einer Verunreinigung der damit gehandhabten Substanzen, der zur Sicherstellung einer hohen Produktqualität jedoch nicht ausreichend ist.

FR 2 696 526 A1 offenbart ein Anschlussstück für Fluidleitungen.

Es ist daher die Aufgabe der vorliegenden Erfindung ein Anschlußstück zum Transfer von Fluiden sowie ein entsprechendes Verfahren bereitzustellen, mit dem ein Kontakt zwischen einem in einem mit dem Anschlußstück verbundenen Schlauch oder Behälter enthaltenen Fluid und der Umgebung und insbesondere Personen sowohl während der Dauer der Fluidver-bindung als auch nach der Trennung der Fluidverbindung weitestgehend ausgeschlossen werden kann.

Diese Aufgabe wird gelöst durch ein Anschlußstück gemäß Anspruch 1, eine Verbindungsvorrichtung gemäß Anspruch 15 und ein Verfahren gemäß Anspruch 26. Bevorzugte Ausführungsformen der Erfindung sind in den Unteransprüchen angegeben.

Erfindungsgemäß wird ein Anschlußstück zum abgeschlossenen, tropffreien und sicheren Transfer von Fluiden bereitgestellt, welches eine Öffnung und eine Verschlußeinrichtung zum Verschließen der Öffnung aufweist und zur Herstellung einer dichten Verbindung mit einem weiteren Anschlußstück für den Transfer eines Fluids zwischen den Anschlußstücken eingerichtet ist, und des weiteren einen darin angeordneten Leitungsabschnitt umfaßt, der durch die Öffnung aus dem Anschlußstück vorgeschoben werden kann.

Das erfindungsgemäße Anschlußstück ist verschließbar, so daß ein Austritt eines in einem mit dem Anschlußstück verbundenen Behältnis oder einer Leitung enthaltenen Fluids und des weiteren auch eine Verunreinigung desselben vermieden werden kann. Die eigentliche Verbindung zum Transfer eines Fluids in ein weiteres mit dem Anschlußstück verbindbares Anschlußstück oder Behältnis wird durch einen im Anschlußstück beweglich angeordneten Leitungsabschnitt hergestellt, der aus dem Anschlußstück in ein damit verbundenes zweites Anschlußstück oder sonstiges Behältnis vorgeschoben werden kann. Vorzugsweise erfolgt die Verbindung mittels des Leitungsabschnitts erst, wenn die Anschlußstücke dicht miteinander verbunden sind und die Verschlußeinrichtung geöffnet wurde. Zur Trennung der Verbindung wird vorzugsweise zunächst der bewegliche Leitungsabschnitt in das eine Anschlußstück zurückgeführt, danach die Verschlußeinrichtung geschlossen und wird das Anschlußstück erst dann von dem damit verbundenen Anschlußstück oder Behälter getrennt, so daß weder Fluid, das sich beispielsweise noch im beweglichen Leitungsabschnitt befindet, austreten, noch eine Verunreinigung des Fluids durch die Umgebung erfolgen kann.

Bei der erfindungsgemäßen Vorrichtung ist somit ein Austritt von sich im Leitungsabschnitt befindendem Restfluid, beispielsweise durch einen Überdruck im Leitungsabschnitt, oder ein Anhaften von Fluid an der Außenseite des Leitungsabschnitts weitestgehend ausgeschlossen, da der Leitungsabschnitt aufgrund der Verschlußeinrichtung sowohl vor dem Verbinden mit einem weiteren Anschlußstück als auch im verbundenen Zustand der Anschlußstücke sowie nach dem Trennen der Anschlußstücke nicht zugänglich ist. Umgekehrt ist das Fluid gegenüber Verunreinigungen durch die Umgebung geschützt läßt sich auch für aseptische Zwecke einsetzen.

Gemäß einer Ausführungsform ist die Verschlußeinrichtung so eingerichtet, daß ein Leitungsabschnitt durch diese vorgeschoben und daraus zurückgezogen werden kann, wobei die Verschlußeinrichtung zusätzlich eine Dichtungsfunktion umfaßt, so daß das Anschlußstück gegenüber dem Leitungsabschnitt abgedichtet ist, während der Leitungsabschnitt durch die Verschlußeinrichtung vorgeschoben oder zurückgezogen wird.

Mit einer derartig ausgebildeten Verschlußeinrichtung bleibt das Anschlußstück auch bei vorgeschobenem Leitungsabschnitt durch die Verschlußeinrichtung zur Außenseite abgedichtet und Fluid kann aus dem Anschlußstück ausschließlich durch den Leitungsabschnitt austreten. Darüber hinaus hat die Dichtungsfunktion zwischen dem Leitungsabschnitt und der Verschlußeinrichtung zusätzlich die Aufgabe, am Leitungsabschnitt außen anhaftendes Fluid beim Zurückziehen des Leitungsabschnitts durch die Verschlußeinrichtung abzustreifen, so daß es somit nicht in das Anschlußstück bzw. aus diesem befördert werden kann. Einer weiteren Ausführungsform entsprechend umfaßt das Anschlußstück im Inneren einen verschiebbaren Halter, an dem der Leitungsabschnitt angeordnet ist. Gemäß einer Ausfühmngsform ist die Verschlußeinrichtung eine Membran oder Septum und der Leitungsabschnitt eine Nadel. Denkbar ist jedoch auch eine Ausbildung der Verschlußeinrichtung als eine mechanische, einem optischen Zentralverschluß ähnliche Verschlußeinrichtung oder eine sonstige, beispielsweise schwenkbare oder klappbare Verschlußeinrichtung.

Das Anschlußstück kann gemäß einer Ausführungsform eine Sperrklinke umfassen, wobei mit der Sperrklinke ein Verschieben des Halters verhindert wird, wenn das Anschlußstück von dem weiteren Anschlußstück getrennt ist. Damit kann eine Verletzungsgefahr durch ein Vorschieben der Nadel aus dem Anschlußstück, wenn dieses nicht mit einem anderen Anschlußstück verbunden ist, vermieden werden.

Erfindungsgemäß wird des weiteren ein Anschlußstück zum abgeschlossenen, tropffreien, sicheren und kontaminationsfreien Transfer von Fluiden bereitgestellt, welches zum Zusammenwirken mit einem oben beschriebenen erfindungsgemäßen Anschlußstück mit einem darin angeordneten beweglichen Leitungsabschnitt und zur Aufnahme des beweglichen Leitungsabschnitts zur Herstellung einer Fluidverbindung eingerichtet ist.

Gemäß einer bevorzugten Ausführungsform kann das zweite Anschlußstück ebenfalls eine Verschlußeinrichtung zum Verschließen der Öffnung aufweisen, die so eingerichtet ist, daß ein Leitungsabschnitt durch diese in das Anschlußstück vorgeschoben und daraus zurückgezogen werden kann, wobei auch diese Verschlußeinrichtung eine Dichtungsfunktion umfassen kann, durch die das Anschlußstück gegenüber dem Leitungsabschnitt abgedichtet ist, wenn der Leitungsabschnitt durch die Verschlußeinrichtung durch das Anschlußstück vorgeschoben oder zurückgezogen wird.

Gemäß einer weiteren bevorzugten Ausführungsform umfaßt das zweite Anschlußstück einen darin fest angeordneten Leitungsabschnitt, der mit dem beweglichen Leitungsabschnitt des ersten Anschlußstücks zur Herstellung einer vorzugsweise nach außen abgedichteten Fluidtransferleitung verbunden werden kann.

Durch Bereitstellen von Verschlußeinrichtungen an beiden Anschlußstücken kann eine besonders hohe Sicherheit gegen Kontaminationen der Außenumgebung durch ein in einem Anschlußstück beinhaltetes Fluid sowie gegen Verunreinigungen eines Fluids durch Substanzen aus der Umgebung gewährleistet werden, da jedes Anschlußstück für sich verschlossen ist und ein Austritt von Fluid oder ein Eindringen von verunreinigenden, die Produktqualität vermindernden Substanzen auch bei getrennten Anschlußstücken vermieden werden kann. Die Dichtungsfunktion der Verschlußeinrichtungen gegenüber dem Leitungsabschnitt trägt zusätzlich dazu bei, daß weder bei einem Vorschieben des Leitungsabschnitts von einem in das andere Anschlußstück noch in umgekehrter Richtung an der Außenwand des Leitungsabschnitts anhaftendes Fluid aus einem der Anschlußstücke zur Außenseite gelangen kann und somit auch nach der Trennung der Anschlußstücke deren Außenflächen frei von Fluidresten sind.

Gemäß noch einer weiteren Ausführungsform bildet die Verschlußeinrichtung, die für ein Zusammenwirken mit einer vorzugsweise baugleichen Verschlußeinrichtung eines weiteren Anschlußstücks eingerichtet ist, gleichzeitig eine Einrichtung zur Herstellung einer dichten Verbindung zwischen den Öffnungen zweier Anschlußstücke. Dazu kann die Verschlußeinrichtung gemäß einer Ausführungsform als eine Membran oder ein Septum ausgebildet sein, die die Öffnung eines Anschlußstücks überdeckt und beim Zusammenfügen mit einem anderen Anschlußstücke auf einer Membran desselben zur Anlage kommt.

Die Gefahr eines Austritts von Fluid sowie einer Verunreinigung von Fluid kann damit weiter reduziert werden, da zusätzlich zum gegenüber dem Anschlußstück isolierten Leitungsabschnitt eine weitere, durch die miteinander zusammenwirkenden Verschlußeinrichtungen realisierte Dichtungseinrichtung vorgesehen wird, die vorzugsweise beim Zusammenfügen der Anschlußstücke und somit bevor der Leitungsabschnitt zum Übertragen des Fluids in das zweite Anschlußstück vorgeschoben wird, eingerichtet wird. Gemäß einer alternativen Ausführungsform könnte die zusätzliche durch Zusammenwirken der Verschlußeinrichtungen hergestellte Dichtung auch als separate Einrichtung, wie beispielsweise als ein Dichtungsring an einem oder beiden Anschlußstücken vorgesehen sein.

Gemäß einer vorteilhaften Ausführungsform ist die Membran beweglich am Anschlußstück angeordnet, so daß die Stelle an der Membran, an der der Leitungsabschnitt durch diese vorgeschoben wird, verändert werden kann. Dazu ist die Membran vorteilhafter Weise drehbar am Anschlußstück angeordnet. Die Membran könnte alternativ auch verschiebbar angeordnet sein. Beim Durchstoßen der Membran bzw. des Septum durch den z.B. als Nadel ausgebildeten Leitungsabschnitt, wird in der Membran eine Öffnung erzeugt, die sich aufgrund der Elastizität des Materials der Membran beim Zurückziehen des Leitungsabschnitts von selbst wieder verschließt. Ein wiederholter Gebrauch kann jedoch zu Verschleißerscheinungen und zu einer verminderten Dichtigkeit der Membran führen, die eine Erneuerung der Membran erfordern kann. Mit Hilfe einer so drehbar angeordneten Membran, daß ihre Drehachse gegenüber der Längsachse der Nadel versetzt ist, kann der Ort des Durchstoßes der Nadel durch die Membran verändert und somit die Lebensdauer der Membran vorteilhaft erhöht werden. Eine entsprechende Vorrichtung zum Verschieben oder Drehen der Membran könnte automatisch gesteuert sein, so daß die Membran beispielsweise nach einer bestimmten Anzahl von Verwendungen des Anschlußstücks automatisch um eine bestimmte Strecke verschoben oder gedreht wird. Eine einfachere Ausführung kann eine manuell verschiebbare oder drehbare Membran umfassen, die gegebenenfalls Rastpositionen umfaßt, durch die bestimmte Strekken, um die die Membran bewegt werden kann, vorgegeben werden.

Gemäß einer noch weiteren Ausführungsform umfaßt das Anschlußstück eine Kupplungseinrichtung für eine feste, lösbare Verbindung mit einem weiteren Anschlußstück. Dadurch wird ein unbeabsichtigtes Lösen der Verbindung und insbesondere während eines Transfers von Fluiden verhindert. Die Kupplungseinrichtung kann beispielsweise als Schraubverbindung, insbesondere als Luer-Lock, oder Bajonettverschluß oder als eine sonstige geeignete, gegebenenfalls automatisch einrastende oder verriegelnde Verbindungseinrichtung ausgeführt sein.

Gemäß noch einer weiteren Ausführungsform umfaßt das Anschlußstück eine Einrichtung oder einen Teil einer Einrichtung, die eine Trennung des Anschlußstücks von einem damit verbundenen weiteren Anschlußstück oder das Zusanunenfügen oder die Kupplung mit einem weiteren Anschlußstück nur zuläßt, wenn der bewegliche Leitungsabschnitt in das Anschlußstück zurückgezogen ist.

Damit ist ein Trennen zweier miteinander verbundener Anschlußstücke nur möglich, wenn der Leitungsabschnitt in eines der Anschlußstücke und insbesondere ohne von außen zugänglich zu sein, hinter die Verschlußeinrichtung zurückgezogen ist, so daß ein Austritt von Fluid aus einem der Anschlußstücke oder eine Kontamination durch sich im Leitungsabschnitt befindendes oder an der Außenseite des Leitungsabschnitts anhaftendes Fluid vermieden werden kann. Da die Anschlußstücke nicht voneinander getrennt werden können, während der Leitungsabschnitt aus einem der Anschlußstücke vorgeschoben ist, kann auch die Verletzungsgefahr durch einen als Nadel ausgebildeten Leitungsabschnitt im wesentlichen ausgeschlossen und eine Verunreinigung von Fluid in den Anschlußstücken vermieden werden.

Gemäß noch einer weiteren Ausführungsform ist das Anschlußstück als Stecker oder als Buchse ausgebildet, wobei der Stecker bzw. die Buchse eine Einrichtung aufweist, die für ein Zusammenfügen oder für eine Verriegelung mit einer entsprechenden Einrichtung an der Buchse bzw. am Stecker eingerichtet ist. Mit Hilfe geeigneter zusammenpassender Einfügeeinrichtungen an Stecker und Buchse kann ein einfaches, zu einer festsitzenden Verbindung führendes Zusammenfügen der Anschlußstücke und eine Ausrichtung der Öffnungen der Anschlußstücke sowie des beweglichen Leitungsabschnitts im Verhältnis zum Anschlußstück sichergestellt werden.

Gemäß noch einer weiteren Ausführungsform umfaßt das Anschlußstück einen Leitungsabschnitt mit einem Durchmesser von weniger als 5 mm, bevorzugt von weniger als 2 mm und besonders bevorzugt von weniger als 1 mm. Der Außendurchmesser des Leitungsabschnitts ist vorzugsweise kleiner als die Öffnung des Anschlußstücks, so daß beim Vorschieben des Leitungsabschnitts aus dem Anschlußstück kein Kontakt mit dieser besteht. Vorzugsweise ist der Außendurchmesser des Leitungsabschnitts wesentlich kleiner als der der Öffnung gewählt.

Das erfindungsgemäße Anschlußstück eignet sich für alle Arten von Fluiden. Insbesondere ist es für den Transfer von medizinischen und insbesondere radioaktive Substanzen enthaltenden Substanzverbindungen und Medikamenten eingerichtet.

Erfindungsgemäß wird des weiteren eine Verbindungsvorrichtung zum abgeschlossenen, tropffreien, sicheren und kontaminationsfreien Transfer von Fluiden mit zwei miteinander verbindbaren Anschlußstücken bereitgestellt, die eingerichtet sind, im miteinander verbundenen Zustand eine dichte Verbindung zwischen den Öffnungen der Anschlußstücke herzustellen, wobei eines der Anschlußstücke eine Öffnung und eine Verschlußeinrichtung zum Verschließen der Öffnung und einen darin angeordneten beweglichen Leitungsabschnitt umfaßt, der im verbundenen Zustand der Anschlußstücke durch die Öffnung aus dem einen Anschlußstück in das andere Anschlußstück zur Herstellung einer Fluidverbindung vorgeschoben werden kann.

Die erfindungsgemäße Verbindungsvorrichtung gewährleistet eine sichere Verbindung zum Transfer von Fluiden zwischen zwei Anschlußstücken, da zunächst eine Verbindung zwischen den Anschlußstücken durch mechanisches Verbinden derselben hergestellt werden kann, während als eigentliche Leitung zum Transferieren eines Fluids der bewegliche Leitungsabschnitt aus dem einen Anschlußstück in das zweite Anschlußstück vorgeschoben wird. Eine Kontamination durch Fluid im oder an der Außenseite des zurückgezogenen Leitungsabschnitts kann durch eine am Anschlußstück vorgesehene Verschlußeinrichtung vermieden werden. Des weiteren kann mit der erfindungsgemäßen Verbindungsvorrichtung auch eine hohe Produktqualität sichergestellt werden, da eine Verunreinigung eines Fluids durch Substanzen aus der Umgebung vermieden werden kann. Die erfindungsgemäße Verbindungsvorrichtung kann auch für aseptische Zwecke verwendet werden.

Gemäß einer bevorzugten Ausführungsform ist die Verschlußeinrichtung so eingerichtet, daß ein Leitungsabschnitt durch diese vorgeschoben und daraus zurückgezogen werden kann, wobei die Verschlußeinrichtung zusätzlich eine Dichtungsfunktion umfaßt, so daß das Anschlußstück gegenüber dem Leitungsabschnitt während des Vorschiebens oder Zurückziehens des Leitungsabschnitts und auch im vorgeschobenen Zustand des Leitungsabschnitts durch die Verschlußeinrichtung abgedichtet ist.

Die Dichtungsfunktion der Verschlußeinrichtung hat darüber hinaus die Wirkung, daß beim Vorschieben oder Zurückziehen des Leitungsabschnitts an der Außenseite des Leitungsabschnitts anhaftendes Fluid abgestreift wird und nicht aus dem Anschlußstück zur Außenseite gelangen kann. Die Verschlußeinrichtung kann beispielsweise durch eine auf der Öffnung des Anschlußstücks angeordnete Membran oder ein Septum realisiert sein. Des weiteren kann vorgesehen werden, daß die Verbindungsvorrichtung eine Verschlußeinrichtung an jedem der Anschlußstücke umfaßt und die Verschlußeinrichtungen in Zusammenwirkung zur Realisierung einer dichten Verbindung zwischen den Öffnungen der Anschlußstücke für den Transfer eines Fluids zwischen den Anschlußstücken eingerichtet sind. Diese Verschlußeinrichtung kann mit Hilfe von zwei an den Anschlußstücken angeordneten und beim Verbinden der Anschlußstücke aufeinander gepreßten Membranen oder Septa realisiert sein.

Dadurch kann sichergestellt werden, daß weder Fluid nach außen entweichen noch eine Verunreinigung des Fluids erfolgen kann, wenn die Anschlußstücke miteinander verbunden sind. Alternativ könnte die gemäß dieser Ausführungsform vorgesehene Dichtungsfunktion der Verschlußeinrichtungen jedoch auch durch eine separate an einem oder an beiden Anschlußstücken vorgesehene Dichtungseinrichtung, wie beispielsweise einen Dichtungsring, realisiert werden.

Gemäß noch einer weiteren Ausführungsform umfaßt das Anschlußstück ohne den beweglichen Leitungsabschnitt einen festen Leitungsabschnitt, mit dem der bewegliche Leitungsabschnitt zur Herstellung einer Fluidverbindung in Kontakt gebracht werden kann. Dazu weisen die Leitungsabschnitte vorzugsweise Durchmesser auf, die ein Einfügen ineinander zulassen, d.h. der Außendurchmesser des einen Leitungsabschnittes entspricht dem Innendurchmesser des anderen Leitungsabschnitts. Alternativ oder zusätzlich kann an einem oder an beiden Leitungsabschnitten eine zusätzliche Dichtungseinrichtung vorgesehen sein.

Gemäß noch einer weiteren Ausführungsform umfassen die Anschlußstücke Einrichtungen, die eine Kupplung der Anschlußstücke oder eine Trennung der gekoppelten Anschlußstücke nur zulassen, wenn der bewegliche Leitungsabschnitt in das Anschlußstück zurückgezogen ist. Dadurch kann eine unbeabsichtigte Trennung der Verbindung zwischen den Anschlußstücken, während der Leitungsabschnitt aus dem einen Anschlußstück in das andere vorgeschoben ist, vermieden werden, wobei auch eine Kontamination durch gegebenenfalls an der Außenseite des Leitungsabschnitts anhaftendes oder darin vorhandenes Fluid oder eine Verletzung durch den Leitungsabschnitt ausgeschlossen wird.

Gemäß noch einer weiteren Ausführungsform ist eines der Anschlußstücke als Stecker und das andere Anschlußstück als eine mit dem Stecker verbindbare oder kuppelbare Buchse ausgebildet, wobei Stecker und Buchse so konfiguriert sind, daß sich die Öffnungen des Steckers und der Buchse im zusammengefügten Zustand gegenüberliegen. Durch entsprechende Formgebung von Stecker und Buchse wird ein rasches Zusammenfügen der Anschlußstücke sichergestellt.

Gemäß noch einer bevorzugten Ausfühmngsform umfassen die Anschlußstücke jeweils eine mit einer Membran als Verschlußeinrichtung verschlossene Öffnung. Die Verwendung einer Membran hat den Vorteil, daß sie kostengünstig ist und gleichzeitig als Verschlußeinrichtung sowie als Dichtungseinrichtung verwendet werden kann. Wird an der Öffnung jedes Anschlußstücks eine Membran vorgesehen, kann durch eine Anlage der Membranen aneinander, die beispielsweise aus Gummi hergestellt sein können, eine dichte Verbindung zwischen den Anschlußstücken erreicht werden, die auch bei vorgeschobenem Leitungsabschnitt bestehen bleibt. Durch festes Aufeinanderpressen der Membranen ist darüber hinaus eine Kontamination der auf der Außenseite der Anschlußstücke liegenden Membranoberflächen im wesentlichen ausgeschlossen. Das Öffnen und Schließen der Verschlußeinrichtung wird durch Durchstoßen der Membran mit einer spitzen Nadel ausgeführt. Die Membran bildet darüber hinaus eine Dichtungseinrichtung gegenüber dem Leitungsabschnitt, wobei an dem Leitungsabschnitt anhaftendes Fluid beim Zurückziehen der Nadel im Anschlußstück zurückgehalten wird.

Gemäß einer Ausführung kann der Leitungsabschnitt eine konzentrisch zur Öffnung des Anschlußstücks angeordnete Nadel sein, wobei im verbundenen Zustand der Anschlußstücke die Nadel durch beide Membranen vorgeschoben bzw. zurückgezogen werden kann.

Der Leitungsabschnitt kann beispielsweise einen Durchmesser von weniger als 5 mm, bevorzugt von weniger als 2 mm und besonders bevorzugt von weniger als 1 mm umfassen.

Des weiteren können die Anschlußstücke eine zylindrische Form und die Membranen eine kreisrunde Form aufweisen und die Membranen zusammen mit dem Leitungsabschnitt jeweils konzentrisch zur Längsachse der Anschlußstücke im verbundenen Zustand oder deren Öffnungen angeordnet sein.

Gemäß einer weiteren vorteilhaften Ausführungsform ist zumindest eine Membran beweglich am Anschlußstück angeordnet, daß die Stelle an der Membran, an der der Leitungsabschnitt durch diese vorgeschoben wird, verändert werden kann. Dazu ist die Membran vorteilhafter Weise drehbar am Anschlußstück angeordnet. Alternativ könnte sie auch verschiebbar angeordnet sein. Das wiederholte Durchstoßen der Membran bzw. des Septums durch den z.B. als Nadel ausgebildeten Leitungsabschnitt kann zu Verschleißerscheinungen und einer verminderten Dichtigkeit der Membran führen. Durch eine manuell oder automatisch so verschiebbar oder drehbar angeordnete Membran, daß der Ort des Durchstoßes durch die Membran verändert werden kann, kann die Lebensdauer der Membran vorteilhaft erhöht werden.

Erfindungsgemäß wird des weiteren ein Verfahren zur Herstellung einer Fluidleitung zwischen zwei Anschlußstücken bereitgestellt, wobei zumindest eines eine mit einer Verschlußeinrichtung verschlossene Öffnung und eines der Anschlußstücke einen bewegbaren Leitungsabschnitt umfaßt, wobei das Verfahren die Schritte eines Zusammenfügens der Anschlußstücke und Herstellen einer dichten Verbindung zwischen diesen und Vorschiebens des Leitungsabschnitts von einem Anschlußstück durch die Öffnungen beider Anschlußstücke in das andere Anschlußstück umfaßt. Gemäß einer Ausführungsform kann es sich bei der Verschlußeinrichtung um eine Membran und bei dem bewegbaren Leitungsabschnitt um eine Nadel handeln, die durch die Membran zwischen den miteinander verbundenen Anschlußstücken geführt wird. Das erfindungsgemäße Verfahren eignet sich zum abgeschlossenen, tropffreien und sicheren Transfer von Fluiden und für aseptische Verwendungen. Es kann damit sowohl eine hohe Sicherheit sowohl gegen einen Kontakt mit Personen durch gefährliche Substanzen als auch die Aufrechterhaltung einer hohen Produktqualität gewährleistet werden.

Im Folgenden wird die Erfindung anhand einer beispielhaften Ausführungsform unter Bezugnahme auf die beigefügten Zeichnungen beschrieben.

In den Zeichnungen zeigt:
- Fig. 1: schematisch eine zwei Anschlußstücke umfassende Verbindungsvorrichtung im Querschnitt gemäß einer Ausführungsform im nicht verbundenen Zustand;
- Fig. 2: einen Ausschnitt der in Fig. 1 gezeigten Verbindungsvorrichtung in einer Phase während des Verbindens der Anschlußstücke; und
- Fig. 3: die Verbindungsvorrichtung gemäß der Ausführungsform mit den Anschluß- stücken im verbundenen Zustand.

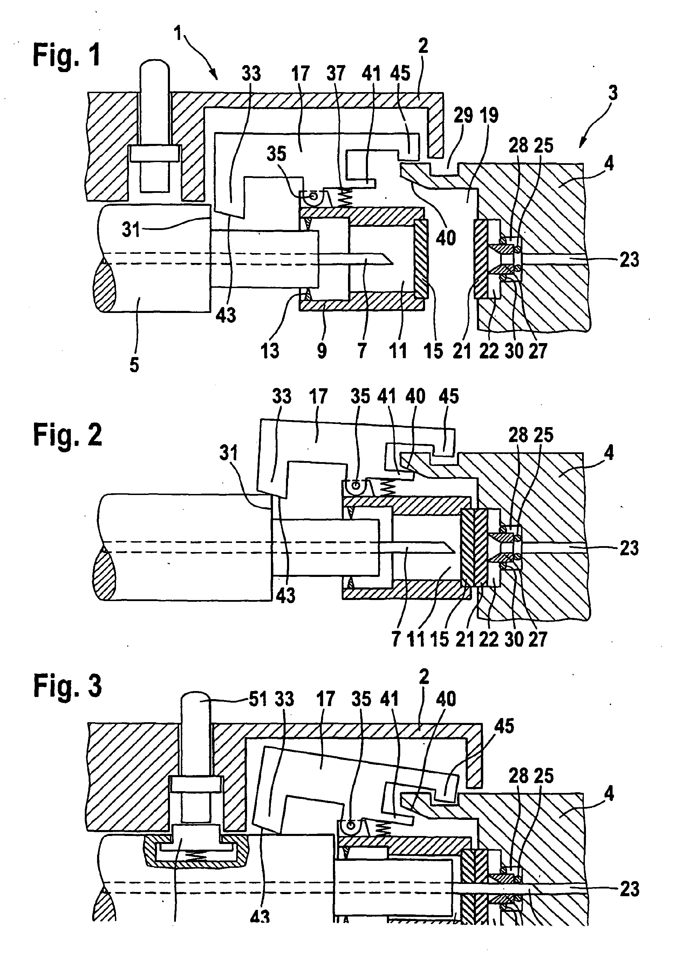

Im Folgenden wird auf die Figuren 1 bis 3 Bezug genommen. Wie in den Figuren gezeigt ist, umfaßt die erfindungsgemäße Verbindungsvorrichtung ein als Stecker 1 ausgebildetes erstes Anschlußstück mit einem Steckergehäuse 2 sowie ein als Buchse 3 ausgebildetes zweites Anschlußstück. In der Zeichnung ist nur der obere Teil des Steckers 1 und der Buchse gezeigt, während das Steckergehäuse 2 tatsächlich den gesamten Stecker 1 umschließt. An den Stecker und die Buchse kann sich ein Behälter, Schlauch, Katheter, eine medizinische Vorrichtung etc. anschließen und der Stecker und die Buchse können fest in eine derartige Vorrichtung integriert sein. Im Innern des Steckers 1 ist ein an einem verschiebbaren Nadelhalter 5 angeordneter Leitungsabschnitt bzw. Nadel 7 vorgesehen. Der Stecker 1 umfaßt des weiteren im Inneren einen Gehäuseabschnitt 9, der in einer Öffnung 11 mündet, in dem der Nadelhalter 5 verschiebbar angeordnet ist und der diesen umgibt. Der Gehäuseabschnitt 9 dient als Führung für den Nadelhalter 5 und umfaßt einen Innendurchmesser, der dem Außendurchmesser des Nadelhalters entspricht. Der Nadelhalter 5 mit der daran angeordneten Nadel 7 kann zwischen einer zurückgezogenen Position, wie sie in Fig. 1 gezeigt ist, und einer vorgeschobenen Position bewegt werden, die in Fig. 3 dargestellt ist. Der Nadelhalter 5 ist gegenüber dem Gehäuseabschnitt 9 mit Hilfe einer Dichtung, wie z.B. eines Dichtungsrings 13, abgedichtet. Des weiteren ist an der Öffnung 11 eine diese verschließende Membran 15 vorgesehen. Die Membran ist vorzugsweise als ein aus Gummi hergestelltes Septum ausgebildet. Des weiteren umfaßt der Stecker 1 eine Sperrklinke 17, mit der ein Verschieben des Nadelhalters 5 verhindert wird, wenn der Stecker 1 nicht mit der Buchse 3 verbunden ist. Die Funktionsweise der Sperrklinke 17 wird nachfolgend in Einzelheiten erläutert.

Die in der Zeichnung ebenfalls nur teilweise dargestellte Buchse 3 weist eine als Vertiefung ausgebildete Aufnahme 19 für den vom Stecker 1 vorragenden Gehäuseabschnitt 9 auf. In der Aufnahme 19 ist eine der am Stecker 1 vorgesehenen Membran 15 bezüglich des Aufbaus und der Anordnung entsprechende Membran 21 vorgesehen, die eine Öffnung 22 der Buchse 3 verschließt. Hinter der Membran 21 ist in der Buchse 3 ein fest darin angeordneter Leitungsabschnitt 23 vorgesehen, der mit Hilfe eines Dichtungsrings 25 gegenüber der Buchse 3 abgedichtet ist. Die Buchse 3 umfaßt des weiteren eine vor und konzentrisch zum Leitungsabschnitt 23 angeordnete Einführhilfe 27 für die bewegliche Nadel 7 des Steckers 1, die in einer entsprechenden Aussparung 28 der Buchse aufgenommen ist. Auch die Einführhilfe 27 ist mit Hilfe eines Dichtungsrings 30 gegenüber der Buchse 3 abgedichtet. Entlang des äußeren Umfangs des Buchsengehäuses 4 der Buchse 3 ist des weiteren eine Sperrnut 29 für ein Zusammenwirkung mit der Sperrklinke 17 vorgesehen, deren Funktion und Wirkungsweise in Verbindung mit der Sperrklinke 17 im folgenden beschrieben wird.

Beide Anschlußstücke 1, 3 sind im voneinander getrennten Zustand gegenüber der Außenumgebung abgedichtet, so daß weder Fluid aus den Anschlußstücken nach außen dringen kann noch Verunreinigungen des Fluids über das Anschlußstück erfolgen können. Somit kann eine hohe Produktqualität sichergestellt werden.

Das Steckergehäuse 2, der Nadelhalter 5 und das Buchsengehäuse 4 umfassen vorzugsweise eine zylindrische, symmetrische Form und die Öffnung 11, Membran 15, der Nadelhalter 5 und die Nadel 7 sind konzentrisch zur Achse des Steckers 1 angeordnet, während die Membran 21, der Leitungsabschnitt 23 und die Einführhilfe 27 konzentrisch zur Längsachse der Buchse 3 angeordnet sind. Das Steckergehäuse 2 und das Buchsengehäuse 4 können ohne weiteres auch eine andere Form aufweisen und beispielsweise quaderförmig ausgebildet sein. Der Stecker 1 und die Buchse 3 oder Teile davon können aus Kunststoff, wie beispielsweise ABS, PI, PE oder PP oder Metall oder aus jedem anderen geeigneten Material hergestellt sein.

Das Verbinden bzw. Trennen der Anschlußstücke wird anhand der Figuren 1 bis 3 beschrieben. Wie in Fig. 1 gezeigt ist, befindet sich der Nadelhalter 5 im Stecker 1 anfangs in der zurückgezogenen Position, in der eine Schulter 31 des Nadelhalters 5 an einem ersten Sperrabschnitt 33 der Sperrklinke 17 anliegt und ein Vorschieben des Nadelhalters 5 durch die Sperrklinke 17 verhindert wird. Die Sperrklinke ist um eine Achse 35 schwenkbar gelagert und wird mit Hilfe einer Feder 37 vorgespannt in einer die Vorwärtsbewegung des Nadelhalters 5 sperrenden Position gehalten.

Beim Zusammenfügen von Stecker 1 und Buchse 3 gelangt beim Vorschieben der Buchse 13 in Richtung des Steckers 1 die am Buchsengehäuse 4 vorgesehene abgeschrägte Fläche 40 in Kontakt mit der Nase 41 der Sperrklinke 17, wie in Fig. 2 zu erkennen ist, wodurch die Sperrklinke 17 im Uhrzeigersinn entgegen der Vorspannwirkung der Feder 37 geschwenkt wird. Dadurch wird der Sperrabschnitt 33 der Sperrklinke 17 vom Nadelhalter 5 außer Eingriff gebracht. Die Buchse 3 wird so weit vorgeschoben, bis die Membranen 15 und 21 aufeinander zur Auflage kommen, wie in Fig. 2 gezeigt ist. Durch die gegenseitige Auflage der Membranen 15, 21 wird eine dichte Verbindung zwischen der Öffnung 11 des Steckers 1 und der Öffnung 22 der Buchse 3 erzeugt. In dieser Position können der Stecker 1 und die Buchse 3 mit Hilfe (nicht gezeigter) daran vorgesehener miteinander zusammenwirkender Einrichtungen, wie beispielsweise den Komponenten einer Schraubverbindung, insbesondere Luer-Lock-Verriegelung, oder eines Bajonettverschlusses, aneinander befestigt werden. Eine derartige Einrichtung ist für das Funktionieren der Erfindung jedoch nicht unbedingt notwendig und kann auch weggelassen werden.

Da beim Zusammenfügen von Stecker 1 und Buchse 3 durch die Drehung der Sperrklinke 17 im Uhrzeigersinn der erste Sperrabschnitt 33 über die Schulter 31 des Nadelhalters 5 angehoben wurde, kann der Nadelhalter 5 jetzt in Richtung der Öffnung 11 des Steckers 1 vorgeschoben werden. Beim Vorschieben des Nadelhalters 5 liegt die Schulter 31 an der schrägen Fläche 43 des ersten Sperrabschnitts 33 der Sperrklinke an, wodurch die Sperrklinke beim Vorschieben des Nadelhalters zusätzlich im Uhrzeigersinn gedreht wird, so daß ein zweiter Sperrabschnitt 45 der Sperrklinke 17 in die Sperrnut 29 der Buchse 3 eingreift. Durch das Eingreifen des zweiten Sperrabschnitts 45 der Sperrklinke 17 in die Sperrnut 29 der Buchse 3 wird bei vorgeschobenem Nadelhalter 5 eine Trennung von Stecker 1 und Buchse 3 verhindert.

Der Nadelhalter 5 kann jetzt durch beide Membranen 15, 21 des Steckers 1 und der Buchse 3 in den Leitungsabschnitt 23 der Buchse 3 vorgeschoben werden, bis die Schulter 31 des Nadelhalters 5 am Gehäuseabschnitt 9 des Steckergehäuses 2 anliegt, wie in Fig. 3 gezeigt ist. Die Vorrichtung ist jetzt für einen Transfer eines Fluids vom Stecker 1 in die Buchse 3 oder in umgekehrter Richtung zwischen bereit.

Um eine dichte Verbindung zwischen den Leitungsabschnitten herzustellen wird der Außendurchmesser des Leitungsabschnitts 7 des Steckers 1 dem Innendurchmesser des Leitungsabschnitts 23 der Buchse 3 entsprechend gewählt. Die Dichtigkeit zwischen den Leitungsabschnitten 7, 23 kann mit Hilfe einer an einem der oder beiden Leitungsabschnitten vorgesehenen Dichtungsvorrichtung zusätzlich erhöht werden.

Durch die aneinander anliegenden Membranen 15, 21 wird unabhängig von der Verbindung der Leitungsabschnitte 7, 23 eine dichte Verbindung zwischen dem Steckergehäuse 2 und dem Buchsengehäuse 4 geschaffen, durch welche die Verbindungsvorrichtung nach außen zusätzlich abgedichtet wird. Mit der erfindungsgemäßen Verbindungsvorrichtung kann ein abgeschlossener, tropffreier und sicherer Transfer von Fluiden zwischen zwei Anschlußstükken sichergestellt werden, wobei einerseits ein Kontakt zwischen einem gefährlichen, insbesondere giftigen, ätzenden und/oder radioaktiven Fluid und Personen, die dieses Handhaben, als auch eine Verunreinigung eines Fluids durch gasförmige, flüssige oder feste Substanzen aus der Umgebung vermieden werden kann.

Am Stecker ist des weiteren eine mit einer Feder beaufschlagte Arretierung 49 vorgesehen, die ein unbeabsichtigtes Zurückziehen des Nadelhalters 5 in den Stecker 1 verhindert. Die Arretierung 49 kann durch Drücken auf einen Schalter 51 gelöst werden.

Das Trennen der Anschlußstücke 1, 3 geschieht in umgekehrter Reihenfolge zur Verbindung. Zunächst wird die Arretierung 49 durch Drücken des Schalters 51 gelöst, so daß der Nadelhalter 5 in den Stecker 1 zurückgezogen werden kann. Gegebenenfalls kann ein Mechanismus (z.B. ein Federmechanismus) am Stecker 1 vorgesehen sein, mit dessen Hilfe der Nadelhalter 5 automatisch in das Steckergehäuse 2 zurückfährt. Dabei wird die Nadel durch die beiden Septa zurückgezogen, wobei aufgrund der Dichtwirkung insbesondere zwischen dem an der Buchse 3 vorgesehenen Septum und der Nadel außen an der Nadel anhaftendes Fluid abgestreift wird und in der Buchse 3 zurückbleibt. Beim Zurückziehen der Nadel 7 hinter das Septum des Steckers 1 gleitet der erste Sperrabschnitts 33 der Sperrklinke über die Schulter 31 des Nadelhalters, so daß diese aufgrund der Wirkung der Feder entgegen dem Uhrzeigersinn gedreht wird und der zweite Sperrabschnitt 45 der Sperrklinke 17 von der Sperrnut 29 außer Eingriff gebracht wird. Buchse 3 und Stecker 1 können somit nunmehr voneinander getrennt werden, wobei jedoch zunächst die oben beschriebene zusätzliche Verriegelungseinrichtung zwischen Buchse und Stecker gelöst werden muß.

Da sich die Nadel 7 im Stecker 1 hinter dem Septum befindet, ist eine Kontaminierung der Außenseite des Steckers 1 durch gegebenenfalls in der Nadel verbliebene Fluidtröpfchen ausgeschlossen. Da während der Verbindung des Steckers 1 und der Buchse 3 die beiden Septa aufeinander zur Anlage gebracht wurden, kann eine Kontaminierung der Außenseite der Septa beim Zurückziehen der Nadel ebenfalls vermieden werden.

An der beschriebenen Ausführungsform der Erfindung können zahlreiche Änderungen und Modifizierungen vorgenommen werden, ohne der Umfang der Erfindung zu verlassen. Beispielsweise ist die Realisierung der Sperrklinke bzw. des gesamten Sperrmechanismus auf andere Weise denkbar. Anders als bei der dargestellten Ausführungsform könnte die Sperrklinke ebenso an der Buchse vorgesehen sein. Auch der Nadelhalter könnte anstatt im Stecker 1 an der Buchse realisiert sein. Die beschriebenen Membranen bzw. Septa könnten durch mechanische Verschlußeinrichtungen, wie beispielsweise Zentralverschlußeinrichtungen ersetzt sein. Schließlich sind Stecker bzw. Buchsen denkbar, die nicht alle der beschriebenen Merkmale und Eigenschaften umfassen. Der Stecker könnte beispielsweise anstatt mit der Buchse direkt mit einem Schlauch oder Behälter gekuppelt werden, der keinen Leitungsabschnitt oder Membran umfaßt.

Die in der vorangehenden Beschreibung offenbarten Eigenschaften und Merkmale der Erfindung können einzeln oder in jeder Kombination für die Erfindung von Bedeutung sein.

### Bezugszeichenliste

- 1: Stecker
- 2: Steckergehäuse
- 3: Buchse
- 4: Buchsengehäuse
- 5: Nadelhalter
- 7: Nadel
- 9: Gehäuseabschnitt
- 11: Öffnung
- 13: Dichtungsring
- 15: Membran
- 17: Sperrklinke
- 19: Aufnahme
- 21: Membran
- 22: Öffnung
- 23: Leitungsabschnitt
- 25: Dichtungsring
- 27: Einführhilfe
- 28: Aussparung
- 29: Sperrnut
- 30: Dichtungsring
- 31: Schulter
- 33: Sperrabschnitt
- 35: Achse
- 37: Feder
- 40: Fläche
- 41: Nase
- 43: Fläche
- 45: Sperrabschnitt
- 49: Arretierung

## Patentansprüche

1. Anschlußstück (1) zum abgeschlossenen, tropffreien und sicheren Transfer von Fluiden, welches eine Öffnung (11) und eine Verschlußeinrichtung (15) zum Verschließen der Öffnung (11) aufweist und zur Herstellung einer dichten Verbindung mit einem weiteren Anschlußstück (3) für den Transfer eines Fluids zwischen den Anschlußstücken (1, 3) eingerichtet ist, wobei das Anschlußstück (1) im Inneren einen mit einem Leitungsabschnitt (7) versehenen verschiebbaren Nadelhalter (5) umfaßt, so daß der Leitungsabschnitt (7) durch die Öffnung aus dem Anschlußstück (1) vorgeschoben werden kann, **gekennzeichnet durch**
eine Sperrklinke (17), die um eine Achse schwenkbar gelagert und mittels einer Feder (37) in einer die Vorwärtsbewegung des Nadelhalters (5) sperrenden Position gehalten wird, so daß ein Vorschieben des Nadelhalters (5) verhindert wird, wenn das Anschlußstück (1) vom weiteren Anschlußstück (3) getrennt ist, während **durch** Schwenken der Sperrklinke entgegen der Vorspannung der Feder (37) beim Zusammenfügen mit dem weiteren Anschlußstück (3) die Sperrklinke vom Nadelhalter außer Eingriff gebracht werden kann.

2. Anschlußstück nach Anspruch 1, **dadurch gekennzeichnet, daß** die Sperrklinke (17) einen Abschnitt (33) aufweist, der beim Vorschieben des Nadelhalters (5) mit einer Schulter (31) desselben so zusammenwirkt, daß die Sperrklinke (17) geschwenkt wird, so dass sie in eine Sperrnut (29) des weiteren Anschlußstücks (3) eingreifen kann und eine Trennung der Anschlußstücke bei vorgeschobenem Nadelhalter verhindert.

3. Anschlußstück nach Anspruch 1, **dadurch gekennzeichnet, daß** die Verschlußeinrichtung (15) so eingerichtet ist, daß der Leitungsabschnitt (7) durch diese vorgeschoben und daraus zurückgezogen werden kann, und die Verschlußeinrichtung (15) eine Dichtungsfunktion umfaßt, so daß das Anschlußstück (1) gegenüber dem Leitungsabschnitt (7) abgedichtet ist, wenn der Leitungsabschnitt (7) durch die Verschlußeinrichtung (15) vorgeschoben ist.

4. Anschlußstück (3) zum abgeschlossenen, tropffreien und sicheren Transfer von Fluiden, welches zum Zusammenwirken mit einem weiteren Anschlußstück (1) gemäß Anspruch 1 oder 2 und zur Aufnahme des beweglichen Leitungsabschnittes (7) desselben zur Herstellung einer Fluidverbindung eingerichtet ist, wobei das Anschlußstück (3) einen darin fest angeordneten Leitungsabschnitt (23) umfaßt.

5. Anschlußstück nach Anspruch 4, **dadurch gekennzeichnet, daß** es eine Sperrnut (23) umfasst, in die die Sperrklinke (17) des Anschlußstücks (1) eingreifen kann.

6. Anschlußstück nach Anspruch 4 oder 5, **dadurch gekennzeichnet, daß** es eine Öffnung (22) und eine Verschlußeinrichtung (21) zum Verschließen der Öffnung (22) aufweist, die so eingerichtet ist, daß ein Leitungsabschnitt (7) durch diese in das Anschlußstück (3) vorgeschoben und daraus zurückgezogen werden kann, wobei die Verschlußeinrichtung (21) eine Dichtungsfunktion umfaßt, so daß das Anschlußstück gegenüber dem Leitungsabschnitt (7) abgedichtet ist, wenn der Leitungsabschnitt (7) durch die Verschlußeinrichtung (21) in das Anschlußstück (3) vorgeschoben ist.

7. Anschlußstück nach einem der vorangehenden Ansprüche, **dadurch gekennzeichnet, daß** die Verschlußeinrichtung (15, 21) gleichzeitig eine Einrichtung zur Herstellung einer dichten Verbindung zwischen den Öffnungen zweier Anschlußstücke (1, 3) für den Transfer eines Fluids zwischen den Anschlußstücken (1, 3) bildet, die mit der Verschlußeinrichtung (21, 15) eines weiteren Anschlußstücks (3, 1) zusammenwirkt.

8. Anschlußstück nach einem der vorangehenden Ansprüche, **dadurch gekennzeichnet, daß** die Verschlußeinrichtung eine Membran (15, 21) ist, die so beweglich am Anschlußstück (1, 3) angeordnet ist, so daß die Stelle an der Membran (15, 21), an der der bewegliche Leitungsabschnitt (7) durch diese vorgeschoben wird, verändert werden kann.

9. Anschlußstück nach einem der vorangehenden Ansprüche, **dadurch gekennzeichnet, daß** es eine Kupplungseinrichtung für eine feste, lösbare Verbindung mit einem weiteren Anschlußstück umfaßt.

10. Verbindungsvorrichtung zum abgeschlossenen, tropffreien und sicheren Transfer von Fluiden mit zwei miteinander verbindbaren Anschlußstücken (1, 3), die eingerichtet sind, im miteinander verbundenen Zustand eine dichte Verbindung zwischen Öffnungen (11, 22) der Anschlußstücke (1, 3) herzustellen, wobei eines der Anschlußstücke (1) ein Anschlußstück nach einem der Ansprüche 1 bis 3, 7 bis 9 und das andere Anschlußstück (3) ein Anschlußstück nach einem der Ansprüche 4 bis 9 umfasst.

11. Verbindungsvorrichtung nach Anspruch 10, **dadurch gekennzeichnet, daß** sie eine Verschlußeinrichtung (15, 21) an jedem der Anschlußstücke (1, 3) umfaßt und die Verschlußeinrichtungen (15, 21) in Zusammenwirkung zur Realisierung einer dichten Verbindung zwischen den Öffnungen (11, 22) der Anschlußstücke für den Transfer eines Fluids zwischen den Anschlußstücken (1, 3) eingerichtet sind.

12. Verbindungsvorrichtung nach Anspruch 10 oder 11, **dadurch gekennzeichnet, daß** das Anschlußstück (3) ohne den beweglichen Leitungsabschnitt (7) einen festen Leitungsabschnitt (23) umfaßt, mit dem der bewegliche Leitungsabschnitt (7) zur Herstellung einer Fluidverbindung in Kontakt gebracht werden kann.

13. Verbindungsvorrichtung nach einem der vorangehenden Ansprüche 10 bis 12, **dadurch gekennzeichnet, daß** die Anschlußstücke (1, 3) Einrichtungen (17, 29, 31) umfassen, die eine Kupplung der Anschlußstücke (1, 3) oder eine Trennung der gekuppelten Anschlußstücke (1, 3) nur zulassen, wenn der bewegliche Leitungsabschnitt (7) in das Anschlußstück (1) zurückgezogen ist.

14. Verbindungsvorrichtung nach einem der vorangehenden Ansprüche 10 bis 13, **dadurch gekennzeichnet, daß** die Anschlußstücke (1, 3) jeweils eine mit einer Membran als Verschlußeinrichtung (15, 21) verschlossene Öffnung (11, 22) aufweisen, wobei die Membranen (15, 21) der Anschlußstücke (1, 3) im gekuppelten Zustand der Anschlußstücke (1, 3) aneinander anliegen und **dadurch** eine dichte Verbindung zwischen den Anschlußstücken hergestellt wird.

15. Verfahren zur Herstellung einer Fluidleitung zwischen einem Anschlußstück nach einem der Ansprüche 1 bis 3, 7 bis 9 und einem Anschlußstück (3) nach einem der Ansprüche 4 bis 9, mit den Schritten:
Außer Eingriff bringen der Sperrklinke (17) vom Nadelhalter (5) durch Schwenken der Sperrklinke entgegen der Vorspannung einer Feder (37) beim Zusammenfügen der Anschlußstücke und Herstellen einer dichten Verbindung zwischen den Anschlußstücken (1, 3); und
Vorschieben des Leitungsabschnitts von einem Anschlußstück durch die Öffnungen beider Anschlußstücke in das andere Anschlußstück.

16. Verfahren nach Anspruch 15, **gekennzeichnet durch** Schwenken der Sperrklinke (17) beim Vorschieben des Nadelhalters (5) **durch** in Anlage bringen einer Schulter (31) des Nadelhalters (5) an einem Abschnitt (33) der Sperrklinke (17) und in Eingriff bringen eines Sperrabschnitts (45) der Sperrklinke (17) mit einer Sperrnut (29) des anderen Anschlusstücks (3).

## Claims

1. Connecting piece (1) for closed, drip-free and secure transfer of fluids, which comprises an opening (11) and a sealing device (15) for closing the opening (11) and is arranged to create a sealed connection with a further connecting piece (3) for the transfer of a fluid between the connecting pieces (1, 3), wherein the connecting piece (1) in the interior comprises a mobile needle holder (5) fitted with a pipe section (7) so that the pipe section (7) can be advanced out of the connecting piece (1) through the opening, **characterised by** a locking catch (17) mounted swivelling about an axis and held by means of a spring (37) in a position blocking the forward movement of the needle holder (5) so that advance of the needle holder (5) is prevented when the connecting piece (1) is separated from the further connecting piece (3), while by swivelling the locking catch against the pretension of the spring (37) on assembly with the further connecting piece (3), the locking catch can be brought out of engagement with the needle holder.

2. Connecting piece according to claim 1, **characterised in that** the locking catch (17) has a portion (33) which on advance of the needle holder (5) cooperates with a shoulder (31) thereof such that the locking catch (17) is swivelled so that it can engage in a locking groove (29) of the further connecting piece (3) and prevent separation of the connecting pieces when the needle holder is advanced.

3. Connecting piece according to claim 1, **characterised in that** the sealing device (15) is designed so that the pipe section (7) can be advanced and retracted through this, and the sealing device (15) comprises a sealing function so that the connecting piece (1) is sealed against the pipe section (7) when the pipe section (7) is advanced through the sealing device (15).

4. Connecting piece (3) for closed, drip-free and secure transfer of fluids which is designed to cooperate with a further connecting piece (1) according to claim 1 or 2 and to receive the mobile pipe section (7) thereof to create a fluid connection, wherein the connecting piece (3) comprises a pipe section (23) fixedly arranged therein.

5. Connecting piece according to claim 4, **characterised in that** it comprises a locking groove (23) in which the locking catch (17) of the connecting piece (1) can engage.

6. Connecting piece according to claim 4 or 5, **characterised in that** it has an opening (22) and a sealing device (21) for sealing the opening (22) and designed such that a pipe section (7) can be advanced through this into the connecting piece (3) and retracted therefrom, wherein the sealing device (21) comprises a sealing function so that the connecting piece is sealed against the pipe section (7) when the pipe section (7) is advanced through the sealing device (21) into the connecting piece (3).

7. Connecting piece according to any of the preceding claims, **characterised in that** the sealing device (15, 21) simultaneously forms a device to create a sealed connection between the openings of two connecting pieces (1, 3) for the transfer of a fluid between the connecting pieces (1, 3), which device cooperates with the sealing device (21,15) of a further connecting piece (1, 3).

8. Connecting piece according to any of the preceding claims, **characterised in that** the sealing device is a membrane (15, 21) which is arranged mobile on the connecting piece (1, 3) so that the position at the membrane (15, 21) at which the mobile pipe section (7) is advanced through this can be modified.

9. Connecting piece according to any of the preceding claims, **characterised in that** it comprises a coupling device for a fixed detachable connection with a further connecting piece.

10. Connecting device for closed, drip-free and secure transfer of fluids with two connecting pieces (1, 3) that can be joined together and are designed in the joined state to create a sealed connection between openings (11, 22) of the connecting pieces (1, 3), wherein one of the connecting pieces (1) comprises a connecting piece according to claims 1 to 3, 7 to 9 and the other connecting piece (3) comprises a connecting piece according to any of claims 4 to 9.

11. Connecting device according to claim 10, **characterised in that** it comprises a sealing device (15, 21) on each of the connecting pieces (1, 3) and the sealing devices (15, 21) are designed in cooperation to achieve a sealed connection between the openings (11, 22) of the connecting pieces for the transfer of a fluid between the connecting pieces (1, 3).

12. Connecting device according to claim 10 or 11, **characterised in that** the connecting piece (3) without the mobile pipe section (7) comprises a fixed pipe section (23) with which the mobile pipe section (7) can be brought into contact to create a fluid connection.

13. Connecting device according to any of the preceding claims 10 to 12, **characterised in that** the connecting pieces (1, 3) comprise devices (17, 29, 31) which only allow coupling of the connecting pieces (1, 3) or separation of the coupled connecting pieces (1, 3) when the mobile pipe section (7) is retracted into the connecting piece (1).

14. Connecting device according to any of the preceding claims 10 to 13, **characterised in that** the connecting pieces (1, 3) each have an opening (11, 12) closed with a membrane as a sealing device (15, 21), wherein the membranes (15, 21) of the connecting pieces (1, 3) lie against each other in the coupled state of the connecting pieces (1, 3) and thus a sealed connection is created between the connecting pieces.

15. Method for production of a fluid line between a connecting piece according to any of claims 1 to 3, 7 to 9 and a connecting piece (3) according to any of claims 4 to 9, with the steps:
- bringing the locking catch (17) out of engagement with the needle holder (5) by swivelling the locking catch against the pretension of a spring (37) when joining the connecting pieces, and creating a sealed connection between the connecting pieces (1, 3); and
- advancing the pipe section of one connecting piece through the openings of both connecting pieces into the other connecting piece.

16. Method according to claim 15, **characterised by** swivelling the locking catch (17) on advance of a needle holder (5) by bringing a shoulder (31) of the needle holder (5) to rest on a portion (33) of the locking catch (17) and bringing a locking portion (45) of the locking catch (17) into engagement with a locking groove (29) of the other connecting piece (3).

## Revendications

1. Raccord (1) pour un transfert fermé, sans perte de gouttes et sûr des fluides, lequel comporte un orifice (11) et un dispositif d'obturation (15), destiné à obturer l'orifice (11), et lequel est configuré pour la réalisation d'un assemblage étanche avec un autre raccord (3) pour le transfert d'un fluide entre les raccords (1, 3), ledit raccord (1) comportant à l'intérieur un porte-aiguille (5) mobile en translation, muni d'un tronçon de tube (7), de telle sorte que le tronçon de tube (7) peut être poussé vers l'avant à travers l'orifice hors du raccord (1), **caractérisé par**
un cliquet d'arrêt (17), qui est monté pivotant autour d'un axe et qui est maintenu au moyen d'un ressort (37) dans une position bloquant le mouvement vers l'avant du porte-aiguille (5), de telle sorte qu'un mouvement du porte-aiguille (5) vers l'avant est empêché lorsque le raccord (1) est séparé de l'autre raccord (3), tandis que par le pivotement du cliquet d'arrêt dans le sens opposé à la précontrainte du ressort (37) pendant la jonction avec l'autre raccord (3), le cliquet d'arrêt peut être amené hors de prise du porte-aiguille (5).

2. Raccord selon la revendication 1, **caractérisé en ce que** le cliquet d'arrêt (17) comporte une partie (33) qui, pendant le déplacement du porte-aiguille (5) vers l'avant, coopère avec un épaulement (31) de ce dernier, de telle sorte que le cliquet d'arrêt (17) est amené à pivoter, de telle sorte qu'il peut s'engager dans une rainure d'arrêt (29) de l'autre raccord (3) et empêche une séparation entre les raccords lorsque le porte-aiguille est en position poussée vers l'avant.

3. Raccord selon la revendication 1, **caractérisé en ce que** le dispositif d'obturation (15) est configuré de telle sorte que le tronçon de tube (7) peut être poussé vers l'avant à travers ce dernier et être tiré à nouveau vers l'arrière hors de celui-ci, et le dispositif d'obturation (15) remplit une fonction d'étanchéité, de telle sorte que le raccord (1) est rendu étanche par rapport au tronçon de tube (7) lorsque le tronçon de tube (7) est poussé vers l'avant à travers le dispositif d'obturation (15).

4. Raccord (3) pour le transfert fermé, sans perte de gouttes et sûr des fluides, lequel est configuré pour coopérer avec un autre raccord (1) selon la revendication 1 ou 2 et pour recevoir le tronçon de tube (7) mobile de ce dernier afin d'établir une liaison fluidique, le raccord (3) comprenant un tronçon de tube (23), agencé de manière fixe dans ce dernier.

5. Raccord selon la revendication 4, **caractérisé en ce qu'**il comprend une rainure d'arrêt (29), dans laquelle peut s'engager le cliquet d'arrêt (17) du raccord (1).

6. Raccord selon la revendication 4 ou 5, **caractérisé en ce qu'**il comporte un orifice (22) et un dispositif d'obturation (21) destiné à obturer l'orifice (22), lequel est configuré de telle sorte qu'un tronçon de tube (7) peut être poussé vers l'avant à travers celui-ci dans le raccord (3) et peut à nouveau être tiré hors de celui-ci, le dispositif d'obturation (21) remplissant une fonction d'étanchéité, de telle sorte que le raccord est rendu étanche par rapport au tronçon de tube (7) lorsque le tronçon de tube (7) est poussé vers l'avant à travers le dispositif d'obturation (21) dans le raccord (3).

7. Raccord selon l'une quelconque des revendications précédentes, **caractérisé en ce que** le dispositif d'obturation (15, 21) forme en même temps un dispositif destiné à créer un assemblage étanche entre les orifices de deux raccords (1, 3) pour le transfert d'un fluide entre les raccords (1, 3), lequel coopère avec le dispositif d'obturation (21, 15) d'un autre raccord (3, 1).

8. Raccord selon l'une quelconque des revendications précédentes, **caractérisé en ce que** le dispositif d'obturation est une membrane (15, 21), qui est agencée de manière mobile sur le raccord (1, 3), de telle sorte qu'il est possible de faire varier sur la membrane (15, 21) l'emplacement au niveau duquel le tronçon de tube (7), mobile en translation, est poussé vers l'avant à travers ladite membrane.

9. Raccord selon l'une quelconque des revendications précédentes, **caractérisé en ce qu'**il comprend un dispositif de couplage pour un assemblage ferme amovible avec un autre raccord.

10. Dispositif d'assemblage pour le transfert fermé, sans perte de gouttes et sûr des fluides, comportant deux raccords (1, 3) aptes à être assemblés l'un à l'autre, qui sont configurés pour former, dans la position assemblée l'un à l'autre, un assemblage étanche entre les orifices (11, 22) des raccords (1, 3), l'un des raccords (1) comprenant un raccord selon l'une des revendications 1 à 3, 7 à 9, et l'autre raccord (3) comprenant un raccord selon l'une des revendications 4 à 9.

11. Dispositif d'assemblage selon la revendication 10, **caractérisé en ce qu'**il comprend un dispositif d'obturation (15, 21) sur chacun des raccords (1, 3), et les dispositifs d'obturation (15, 21) sont configurés pour coopérer entre eux en vue de réaliser un assemblage étanche entre les orifices (11, 22) des raccords pour le transfert d'un fluide entre les raccords (1, 3).

12. Dispositif d'assemblage selon la revendication 10 ou 11, **caractérisé en ce que** le raccord (3), sans le tronçon de tube (7) mobile, comprend un tronçon de tube (23) fixe, qui peut être amené en contact avec le tronçon de tube (7) pour créer une liaison fluidique.

13. Dispositif d'assemblage selon l'une quelconque des revendications précédentes 10 à 12, **caractérisé en ce que** les raccords (1, 3) comprennent des dispositifs (17, 29, 31) qui autorisent un couplage des raccords (1, 3) ou une séparation des raccords (1, 3) couplés uniquement lorsque le tronçon de tube (7) mobile est retiré en arrière dans le raccord (1).

14. Dispositif d'assemblage selon l'une quelconque des revendications précédentes 10 à 13, **caractérisé en ce que** les raccords (1, 3) comportent respectivement un orifice (11, 22) obturé par une membrane formant un dispositif d'obturation (15, 21), lesdites membranes (15, 21) des raccords (1, 3) étant en appui l'une contre l'autre dans la position couplée des raccords (1, 3) et, de ce fait, est créé un assemblage étanche entre les raccords.

15. Procédé destiné à la réalisation d'une circulation de fluide entre un raccord selon l'une des revendications 1 à 3, 7 à 9, et un raccord (3) selon l'une des revendications 4 à 9, comportant les étapes :
- désolidarisation entre le cliquet d'arrêt (17) et le porte-aiguille (5) sous l'effet du pivotement du cliquet d'arrêt dans le sens opposé à la précontrainte d'un ressort (37) lors de la jonction des raccords et réalisation d'un assemblage étanche entre les raccords (1, 3) ; et
- déplacement vers l'avant du tronçon de tube d'un raccord à travers les orifices des deux raccords pour pénétrer dans l'autre raccord.

16. Procédé selon la revendication 15, **caractérisé par** le pivotement du cliquet d'arrêt (17) lors du déplacement vers l'avant du porte-aiguille (5) sous l'effet de la mise en appui d'un épaulement (31) du porte-aiguille (5) contre une partie (33) du cliquet d'arrêt (17) et engagement d'une partie de blocage (45) du cliquet d'arrêt (17) dans une rainure d'arrêt (29) de l'autre raccord (3).
